(19) Europäisches Patentamt
European Patent Office
Office européen des brevets

(11) Veröffentlichungsnummer: **0 433 628 A1**

# (12) EUROPÄISCHE PATENTANMELDUNG

(21) Anmeldenummer: **90121165.6**

(51) Int. Cl.[5]: **C07D 493/04**

(22) Anmeldetag: **06.11.90**

(30) Priorität: **17.11.89 DE 3938282**

(43) Veröffentlichungstag der Anmeldung: **26.06.91 Patentblatt 91/26**

(84) Benannte Vertragsstaaten: **DE FR GB IT**

(71) Anmelder: **BAYER AG**
**W-5090 Leverkusen 1 Bayerwerk(DE)**

(72) Erfinder: **Buysch, Hans-Josef, Dr.**
**Brandenburger Strasse 28**
**W-4150 Krefeld(DE)**
Erfinder: **Westeppe, Uwe, Dr.**
**Vogelskamp 72**
**W-4020 Mettmann(DE)**

## (54) Verfahren zur Herstellung von 3,8-Dihydroxy-5a,10b-diphenylcumarano-2',3',2,3-cumaran.

(57) Verfahren zur Herstellung von 3,8-Dihydroxy-5a,10b-diphenylcumarano-2',3',2,3-cumaran (DDCC) durch Kondensation von Resorcin mit Benzil in Gegenwart von sauren Kondensationsmitteln dadurch gekennzeichnet, das die Kondensationsreaktion in Gegenwart von Lösungsmitteln durchgeführt wird, die DDCC in einer Menge von weniger als 12 Gew.-% bei 20 °C lösen, in Gegenwart von polaren Verbindungen, die Resorcin in einer Menge von mindestens 10 Gew.-% bei 20 °C lösen und in Gegenwart von einer starken Säure, die im Reaktionsmedium homogen verteilt werden kann.

EP 0 433 628 A1

# VERFAHREN ZUR HERSTELLUNG VON 3,8-DIHYDROXY-5A,10B-DIPHENYLCUMARANO-2′,3′,2,3-CUMARAN

Es ist bekannt, 3,8-Dihydroxy-5a,10b-diphenylcumarano-2′,3′,2,3-cumaran (DDCC) aus Benzil und Resorcin herzustellen (siehe z.B. Rec. Trav. Chim. 87, 599 (1968)). Die dort beschriebene Kondensationsreaktion wird in Eisessig in Gegenwart von konzentrierter Schwefelsäure durchgeführt. Die Ausbeute beträgt dabei etwa 30 % der Theorie.

Nach der EP-A 314 007 wird in Anwesenheit von sauren Ionenaustauschern auf der Basis von sulfonierten mit Divinylbenzol vernetzten Styrolpolymerisaten gearbeitet. Dadurch kann die Ausbeute zwar auf 58 % der Theorie verbessert werden, das erhaltene Produkt (Schmelzpunkt 254° C) ist aber weniger rein als das aus der oben beschriebenen Kondensationsreaktion mit Schwefelsäure (Schmelzpunkt des Produktes: 262° C).

Der vorliegenden Erfindung liegt die Erkenntnis zugrunde, das man die Ausbeuten bei hoher Reinheit erheblich verbessern kann, wenn man in Gegenwart von Lösungsmitteln arbeitet, die DDCC zu weniger als 12 Gew.-% bei 20° C lösen, in Gegenwart von polaren Verbindungen, die Resorcin zu mindestens 10 Gew.-% bei 20° C lösen und in einer Menge von maximal 50 Gew.-%, bezogen auf 100 Gew.-% des gesamten Lösungsmittelgemisches, vorhanden sind und in Gegenwart von starken Säuren mit einem in Wasser gemessenen pH-Wert von kleiner als 3,1, die im Reaktionsmedium homogen verteilt werden können.

Gegenstand der Erfindung ist somit ein Verfahren zur Herstellung von 3,8-Dihydroxy-5a,10b-diphenylcumarano-2′,3′,2,3-cumaran (DDCC) der Formel I

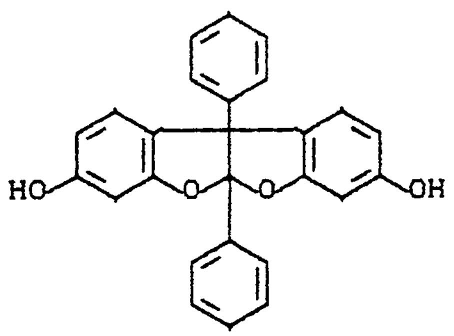

(I)

durch Kondensation von Resorcin mit Benzil in Gegenwart von sauren Kondensationsmitteln, das dadurch gekennzeichnet ist, das man die Kondensation in Gegenwart von

a) Lösungsmitteln, die DDCC zu weniger als 12 Gew.-%, vorzugsweise weniger als 10 Gew.-%, besonders bevorzugt weniger als 8 Gew.-%, ganz besonders bevorzugt weniger als 6 Gew.-% bei 20° C lösen

b) von polaren Verbindungen, die Resorcin zu mindestens 10 Gew.-%, vorzugsweise zu mindestens 15 besonders bevorzugt zu mindestens 30 Gew.-% bei 20° C lösen und in einer Menge von maximal 50 Gew.-%, bevorzugt höchstens 40 Gew.-%, besonders bevorzugt höchstens 35 Gew.-%, bezogen auf 100 Gew.-% des gesamten Lösungsmittelgemisches, vorhanden sind und

c) von starken Säuren, die im Reaktionsmedium homogen verteilt werden können, mit einem in Wasser gemessenen pK-Wert von kleiner als 3,1, bevorzugt kleiner als 2,2, besonders bevorzugt kleiner als 1,6, durchführt.

Ausgangsprodukte für das erfindungsgemäße Verfahren sind Resorcin und Benzil, aber auch die aus vorhergehenden Ansätzen gegebenenfalls isolierten, nicht oder nur teilweise umgesetzten Produkte. Das Molverhältnis von Resorcin zu Benzil beträgt 10:1 bis 0,8:1, vorzugsweise 5:1 bis 1:1. Geeignete Lösungsmittel a) im Sinne der Erfindung sind Kohlenwasserstoffe wie Pentan, Hexan, Heptan, Cyclohexan, Benzol, Toluol, Xylol, Cumol, Diisopropylbenzol, Mesitylen, halogenierte Kohlenwassertoffe wie Dichlormethan, Chloroform, Dichlorethan, Tetrachlorethan, Trichlorethan, Trichlorpropan, Chlorbenzol, Dichlorbenzol, Ether, bevorzugt Ether mit mehr als 4 C- Atomen wie Diisopropylether, Methyl-tert.-butylether, Methyl-tert.-amylether, Anisol und Anethol, Ester, bevorzugt Ester mit mehr als 6 C-Atomen wie Butylpropionat, Isoamylpropionat, Isooctylacetat, Cyclohexylacetat, Alkohole, bevorzugt Alkohole mit mehr als 4 C-Atomen wie Amylalkohol, Isoamylalkohol, Cyclohexylmethanol und 2-Ethylhexanol oder Gemischen dieser Lösungsmittel, vorzugsweise Kohlenwasserstoffe und halogenierte Kohlenwasserstoffe.

Geeignete polare Verbindungen b) im Sinne der Erfindung sind Wasser, wobei auch das Wasser, das während der Kondensation der Reaktionspartner entsteht, in der Regel ausreichende Wirkung entfaltet,

niedere Alkohole wie Methanol, Ethanol, Isopropanol, cyclische Ether wie Tetrahydrofuran, Dioxan, niedere Carbonsäuren wie Ameisensäure, Essigsäure, Propionsäure, Buttersäure, Chloressigsäure, Carbonsäureamide wie Dimethylformamid, Dimethylacetamid, Methylformamid, N-Methylpyrrolidon, N-Methylcaprolactam oder Gemische dieser Verbindungen, bevorzugte Verbindungen b) sind Wasser, niedere Carbonsäuren und niedere Alkohole.

Geeignete starke Säuren c) im Sinne der Erfindung sind durch Substituenten acidifizierte Carbonsäuren wie Dichloressigsäure, Trichloressigsäure, 2,2-Dichlorpropion säure, Trifluoressigsäure, o-Chlorbenzoeaäure, ferner 1-Cyanessigsäure, 1-Cyanpropionsäure, o-Cyanobenzoesäure, o-Nitrobenzoesäure, Malein- und Fumarsäure, Oxalsäure, o-Phthalsäure, Sulfonsäuren wie Methansulfon säure, Trifluormethansulfonsäure, Perfluorbutansulfonsäure, Benzolsulfonsäure, Toluolsulfonsäure, Chlorbenzolsulfonsäure, Phenolsulfonsäure, Sulfanilsäure, Naphthalinsulfonsäure, Benzoldisulfonsäurel Phosphonsäuren wie Methanphosphonsäure, Hydroxymethanphosphonsäure, 1-Hydroxy-ethan-1,1-diphosphonsäure, Benzolphosphonsäure und Benzoldiphosphonsäuren, Mineralsäuren wie Phosphorsäure, Bromwasserstoffsäure, Schwefelsäure und Salzsäure oder Chlorwasserstoff, bevorzugt Salzsäure oder Chlorwasserstoff.

Diese Säuren werden in einer Menge von 1 bis 50 Gew.-%, bevorzugt von 2 bis 30 Gew.-%, besonders bevorzugt von 3 bis 25 Gew.-% bezogen auf das gesamte Reaktionsgemisch zugegeben.

Die Reaktionstemperaturen betragen von 20 bis 120° C, vorzugsweise von 30 bis 110° C, besonders bevorzugt von 30 bis 90° C.

Die Reaktion kann kontinuierlich oder diskontinuierlich durchgeführt werden. Bei der diskontinuierlichen Arbeitsweise legt man das Lösungsmittel, die polare Verbindung und Resorcin vor, dosiert die Säure hinzu und trägt dann unter Rühren Benzil ein. Man kann allerdings auch alle Reaktionskomponenten vorlegen und zuletzt die Säure zugeben. Dabei muß allerdings die Reaktionswärme sehr effizient abgeführt werden können. Daher wird man in einem solchen Fall bevorzugt die kontinuierliche Arbeitsweise wählen. Aber auch hier kann Benzil an verschiedenen Stellen entlang der Reaktionsstrecke zudosiert werden.

Die Reaktionszeit hängt von der Temperatur und der Art der verwendeten Säure ab und liegt zwischen wenigen Minuten und wenigen Stunden. In der Regel ist eine Reaktionsdauer von 0,1 bis 5 Stunden ausreichend, um sehr hohen Umsatz zu erzielen.

Das nach dem erfindungsgemäßen Verfahren erhältliche DDCC kann u.a. zur Herstellung von hochmolekularen Verbindungen, insbesondere von hochmolekularen, thermoplastischen, aromatischen Polycarbonaten gemäß der deutschen Patentanmeldung P 3 934 712,5 verwendet werden.

## Beispiel 1

Ein Gemisch von 743 g (6,7 Mol) Resorcin, 756 g (3.75 Mol) Benzil und 900 g Eisessig und 2000 g Xylol wird mit HCl-Gas begast bei 50-60° C bis zur Sättigung. Insgesamt läßt man 5-6 Stunden bei 50-60° C rühren, drückt das Reaktionsgemisch ab, wäscht mit Xylol mehrmals nach und trocknet bei 100° im Vakuum. Man erhält 1103 g DDCC vom Schmelzpunkt 260-262° C (entsprechend 81 % der Theorie). Aus der Mutterlauge kann weiteres DDCC isoliert werden.

## Beispiel 2

Ein Gemisch von 440 g (4 Mol) Resorcin, 220 g Eisessig und 1040 g Chloroform sättigt man bei 60-65° C mit HCL-Gas und tropft dazu in 3 Stunden eine Lösung von 210 g (1 Mol) Benzil gelöst in 300 g Chloroform und 100 g Eisessig, währenddessen weiter Chlorwasserstoff zugeleitet wird. Nach weiteren 3 Stunden Reaktionszeit wird heiß abgedrückt, mit Chloroform gewaschen und im Vakuum getrocknet. Man erhält 261 g DDCC vom Schmelzpunkt 261-262° C. Beim Ausschütteln der Mutterlauge mit Wasser fallen weitere Kristalle aus, die abgesaugt, mit Chloroform gewaschen und getrocknet werden: Man erhält weitere 95 g, mit dem Schmelzpunkt 258-259° C. Gesamtausbeute: 356 g (entsprechend 90 % der Theorie).

## Beispiel 3

Zu einem bei 60° C mit Chlorwasserstoff begasten Gemisch von 660 g (6 Mol) Resorcin, 2000 g Chloroform und 270 g Eisessig wird in 4 Stunden eine Lösung von 630 g (3 Mol) Benzil in 950 g Chloroform zugetropft. Nach weiteren 3 Stunden Reaktionsdauer wird das Reaktionsprodukt isoliert wie in Beispiel 2 angegeben. Man erhält 931 g vom Schmelzpunkt 259-261° C. Nach Eindampfen der Mutterlauge und Aufkochen des Rückstandes mit Chloroform, Abdrücken, Waschen und Trocknen erhält man weitere 85 g DDCC vom Schmelzpunkt 255-258° C. Gesamtausbeute: 1016 g (entsprechend 85 % der Theorie).

Beispiel 4

Beispiel 2 wird wiederholt, wobei anstelle der Essigsäure 5 g Wasser zugesetzt werden. Man erhält hierbei 363 g DDCC vom Schmelzpunkt 259-260° C (entsprechend 92 % der Theorie) ohne Aufarbeitung der Mutterlauge.

## Ansprüche

Verfahren zur Herstellung von 3,8-Dihydroxy-5a,10b-diphenylcumarano-2',3',2,3-cumaran (Formel I)

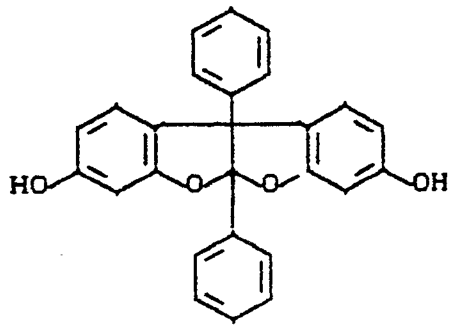

(I)

durch Kondensation von Resorcin mit Benzil in Gegenwart von sauren Kondensationsmitteln, das dadurch gekennzeichnet ist, daß man die Kondensation in Gegenwart

a) von Lösungsmitteln, die DDCC nur in Mengen von weniger als 12 Gew.-% bei 20° C losen, zusätzlich

b) von polaren Verbindungen, die Resorcin zu mindestens 10 Gew.-% bei 20° C losen und in einer Menge von maximal 50 Gew.-%, bezogen auf 100 Gew.-% des gesamten Lösungsmittelgemisches, vorhanden sind und

c) von starken Säuren, die im Reaktionsmedium homogen verteilt werden können, mit einem in Wasser gemessenen $pk_a$-Wert von kleiner als 3,1 durchführt.

Europäisches Patentamt

# EUROPÄISCHER RECHERCHENBERICHT

Nummer der Anmeldung

EP 90121165.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | EP - A1 - 0 314 007 (RÖHM GMBH) * Ansprüche * -- | 1 | C 07 D 493/04 |
| D,A | CHEMICAL ABSTRACTS, Band 69, Nr. 11, 9. September 1968, Columbus, Ohio, USA BUNN, A.; CUDBY, M.E.A.; MC GOWAN, J.C. "Condensation of resorcinol with benzil" Seite 4105, Spalte 1, Zusammenfassung-Nr. 43 822m & Rec. Trav. Chim. Days-Bas 1968, 87(6), 599-608 ---- | 1 | |

RECHERCHIERTE SACHGEBIETE (Int Cl 5)

C 07 D 493/00

Der vorliegende Recherchenbericht wurde für alle Patentansprüche erstellt.

| Recherchenort | Abschlußdatum der Recherche | Prüfer |
|---|---|---|
| WIEN | 30-11-1990 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82